# EUROPEAN PATENT APPLICATION

(11) **EP 2 597 092 A1**
(43) Date of publication of application: **29.05.2013**
(21) Application number: 12194037.3
(22) Date of filing: 23.11.2012
(51) Int. Cl.: C07D 309/30

(54) **A process for purification of lovastatin**

(30) Priority: 24.11.2011 IN MU33212011
(71) Applicant: Sterling Biotech Limited, 400021 Mumbai (IN)
(72) Inventor: Vardhan, Anand, Gujarat (IN); Belwal, Chandrakant, Gujarat (IN); Venkateswarlu, P., Gujarat (IN); Balte, Anup S., Gujarat (IN)
(74) Representative: Kuhnen & Wacker

(57) **Abstract**

The present invention relates to a process for the purification of lovastatin that reduces the level of unknown impurities to below 0.10%. The present invention also discloses a process that is short, less time taking, and industrially viable. The invention also relates to the use of purified lovastatin obtained by the process of the present invention for the synthesis of simvastatin to furnish subsequently pure simvastatin.

## Description

### Field of the Invention

The present invention relates to a process for the purification of lovastatin that reduces the level of unknown impurities to below 0.10%. Also, such a process is short, less time taking, and industrially viable. The invention also relates to the use of purified lovastatin for the synthesis of simvastatin to furnish subsequently pure simvastatin.

### Background of the Invention

Lovastatin is a member of the drug class of statins, used for lowering cholesterol (hypolipidemic agent) in those with hypercholesterolemia and so preventing cardiovascular disease. Lovastatin is an inhibitor of 3-hydroxy-3methylglutaryl-coenzyme A reductase (HMG-CoA reductase), an enzyme that catalyzes the conversion of HMG-CoA to mevalonate.

As the first commercial product of the HMG-CoA reductase inhibitors (statins), Lovastatin is widely used for cholesterol lowering. Meanwhile it is also the key material in the manufacture of simvastatin. In industry, purification of Lovastatin was achieved by several methods one of the purification methods is several times of recrystallization.

Lovastatin of formula (I) known chemically as (1S, 3R, 7S, 8S, 8a R)1,2,3,7,8,8a-hexahydro-3,7-dimethyl-8-[2-[(2R,4R)tetrahydro-4-hydroxy-6-oxo-2H-pyran-2-yl]ethyl]-1-napthalenyl-(S) -2-methylbutyrate is disclosed in U.S. Pat. No. 4,231,938

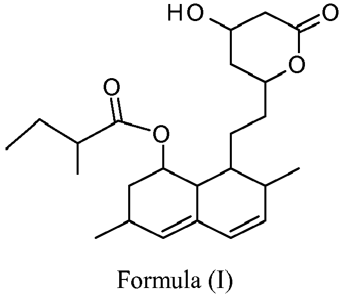

Hypercholesterolemia is known to be a major risk factor for atherosclerosis. Complications of cardiovascular disease, such as myocardial infarction, stroke, and peripheral vascular disease result in many deaths in developed countries. A decrease in plasma LDL levels has been shown to reduce the risk of cardiovascular disease. Statins are by far the most therapeutically effective drugs for reducing the plasma LDL levels. Statins include for example, lovastatin, compactin, pravastatin, simvastatin which are essentially fermentation based and rosuvastatin, fluvastatin and atorvastatin which are totally synthetic statins.

Several processes are reported for recovering lovastatin from fermentation broth which suggest use of different solvents for extraction, recrystallization, use of different chromatographic methods for purification of lovastatin, and the like for example, in U.S. Patent No. 4,916,239; 4,231,938; 5202029; International Publication Nos. (PCT) WO 00/63411 and WO 03/48149.

Several methods are known for isolation of Lovastatin (I) through lactonisation of Mevinolinic acid and purification of impure lovastatin to give pure form of lovastatin, which are summarized herein below:

US Patent No. 4,231,938 (Monaghan R. L et al.) describes a method for purification by column chromatography on silica gel. This method has very little industrial application since it involves purification by chromatography.

PCT Publication No. WO 1997/20834 Al (Dimov I et al.) describes a method for purification of Lovastatin by treating the culture broth with alkaline base in presence of an antioxidant, the filtered and dried raw lovastatin dissolved in a nitrile such as acetonitrile or propionitrile, the solution obtained was decolorized by treating with a mixture of activated carbon and aluminium oxide after addition of water, lovastatin is precipitated at room temperature. This is recrystallized twice successively in a mixture of solvent followed by recrystallization in acetone at -10 to -30°C.

PCT Publication No. WO 2006035295 Al (Kumar et al.) describes a method for purification of Lovastatin by obtaining a solution of lovastatin in toluene, recovering lovastatin, recrystallizing the lovastatin from one or more solvents and recovering the substantially pure lovastatin by the removal of the solvents.

US Patent No. 6,388,100 (Vilmos Keri et al.) describes the process for reducing the dimeric impurities in a statins to less than 0.08% by treatment of statins containing more than 0.08% dimeric impurity with a mild base in a suitable solvent mixture. The mild base includes aliphatic mono, di or tri-amine, aromatic amine, ammonium hydroxide and ammonia. Process requires cooling to -20°C for 24 hours.

Indian Patent No. IN 181828 and IN 181829 describes a method for purification of Lovastatin by recrystallization from methanol or ethanol.

US Patent No. 5,202,029 (Haytko P. N et al.) claims a method for purification of Lovastatin using high performance liquid chromatography (HPLC) using C-18 silica packed HPLC column and eluting with mixture of acetonitrile and water, concentrating the pure fraction to a certain extent and crystallizing by addition of water. This method of purification is not suited for industrial use due to its high cost.

Looking in to cumbersome processes used in the prior art, there is need to develop a simpler, shorter, industrially viable and cost effective purification process which does not involve any chromatographic technique or repeated crystallization.

The present invention provides a purification process that has overcome the drawbacks of the above prior arts.

### Summary of the Invention

It is an object of the present invention to provide a process for purification of lovastatin.

One aspect of the present invention is to provide a process for purification of lovastatin to reduce any unknown impurities to below 0.10%.

The present invention also provides a process for purification of lovastatin that reduces any unknown impurities to below 0.1, and said process is short, less time taking, and industrially viable.

The present invention also provides a process for purification of lovastatin, wherein the process comprises: dissolving lovastatin in a mixture of hydrocarbon and alcohol, treating the solution with activated charcoal, filtering to obtain a filterate, and isolating the pure lovastatin from said filterate by crystallization, and wherein such process reduces any unknown impurities to below 0.10%.

The present invention also provides a process for purification of lovastatin which is short, less time taking, industrially viable and capable of reducing any unknown impurities to below 0.10%, wherein the process comprises:
(i) dissolving lovastatin in a mixture of hydrocarbon and alcohol,
(ii) treating the solution with activated charcoal
(iii) filtering to obtain a filtrate, and
(iv) isolating the pure lovastatin from said filtrate by crystallization.

The present invention also provides a purified lovastatin, wherein any unknown impurities in the compound is below 0.10%.

Another aspect of the invention relates to a method for purification of lovastatin and use of purified lovastatin for the synthesis of simvastatin to furnish subsequently pure simvastatin with good yield.

### Description of the Accompanying Drawings

Fig-1: HPLC chromatogram of pure lovastatin showing unknown impurities below 0.10%
Fig-2: HPLC chromatogram of pure simvastatin prepared from purified lovastatin

### Detailed Description of the Invention

Fermentation process for the synthesis and lactonization process for the production of lovastatin result in the formation of lots of unwanted impurities. These impurities are hard to remove as they crystallize along with the product. There are many methods in prior art to remove these impurities like silica gel column chromatography, high performance liquid chromatography and crystallization process. Crystallization process is most suitable purification process for industrial production of lovastatin but these methods are lengthy, time taking and involve many steps.

The present invention provides a process of purification of lovastatin, which reduces the level of any unknown individual impurities to below 0.10%. The present invention provides a process of purification of lovastatin, which reduces the purification steps to single step. And the present invention provides a process of purification of Lovastatin, which reduces the time of purification process as well.

In present invention the solvent mixture is used to remove the polar as well as non polar impurities in a single crystallization to reduce the purification process time.

The mixture of solvent comprises a hydrocarbon and other component, the hydrocarbon component of the mixture can be toluene, benzene, chlobenzene and other aromatic hydrocarbon. The other component of the solvent mixture can be an alcohol like methanol, ethanol and Isopropanol.

The present invention provides a process of purification of lovastatin which is short, less time taking, industrially viable and capable of reducing any unknown impurities to below 0.10%.

The present invention provides a process for purification of lovastatin to reduce any unknown impurities to below 0.10%, wherein the process comprises:
dissolving lovastatin in a mixture of hydrocarbon and alcohol,
treating the solution with activated charcoal,
filtering to obtain a filtrate, and
isolating the pure lovastatin from said filtrate by crystallization.

The said process for purification of lovastatin is short, less time taking, and is industrially viable.

The hydrocarbon employed in the present invention is an aromatic hydrocarbon. The aromatic hydrocarbon is selected from toluene, benzene and chlorobenzene.

The alcohol employed in the present invention is a C₁-C₄ alcohol. The alcohol is selected from methanol, ethanol and isopropanol.

The mixture of alcohol and hydrocarbon employed in the present invention is in the ratio of 1:9 to 9:1 and preferably 1:9.

The dissolution and activated charcoal treatment steps in the process are carried out at a temperature range of room temperature to 100°C, preferably 50°C to 80°C, and more preferably 65°C to 70°C.

The filtration step in the process is carried out at a temperature range of room temperature to 100°C, preferably 50°C to 80°C, and more preferably 65°C to 70°C.

The crystallization step in the process is carried out at a temperature range of 0°C to 40°C, preferably 0°C to room temperature, and more preferably 0°C to 5°C.

The crystallization step in the process is carried out for a period of 15 minutes to 12 hours, preferably for 3 to 4 hours.

The present invention also provides a process for purification of lovastatin and use of purified lovastatin for the synthesis of simvastatin to furnish subsequently pure simvastatin with good yield.

Comparison of the impurity profile of present invention with that of other purification process is summarized in the table given below

| Parameters | Crystallization solvents or solvent mixture | | | | | | | | |
|---|---|---|---|---|---|---|---|---|---|
| | Present Invention (Solvents used )* | Toluene | MeOH | Toluene followed by MeOH | MIBK | EtOH | IPA | Butanol | Acetone |
| Chromatogra phic purity (%) | 99.62 | 99.18 | 99.26 | 99.39 | 99.23 | 99.26 | 99.30 | 99.46 | 99.10 |
| Impurity-D (Dimmer) (%) | 0.11 | 0.14 | 0.20 | 0.19 | 0.16 | 0.29 | 0.21 | 0.22 | 0.16 |
| Single maximum impurity (%) | 0.09 | 0.24 | 0.14 | 0.14 | 0.15 | 0.16 | 0.18 | 0.09 | 0.20 |

| | | | | | | | | | |
|---|---|---|---|---|---|---|---|---|---|
| * Solvents used Methanol and Toluene MeOH: Methanol EtOH: Ethanol MIBK: Methyl ethyl ketone IPA: Isopropyl alcohol | | | | | | | | | |

### Advantages of the present invention

1. The present invention provides a very short and less time taking process for the purification of lovastatin, which is capable of reducing the unknown impurities to below 0.10%.
2. The present invention provides an industrially viable process for the purification of lovastatin. As lovastatin is more soluble in the mixture of solvent than the individual solvent, the bigger batch size can be taken.
3. The present invention provides a very short and less time taking process for the purification of lovastatin which involves fewer industrial equipments.
4. The present invention does not involve purification by chromatographic techniques.
5. The present invention provides a process for the purification of lovastatin, which can be used for the synthesis of subsequently pure simvastatin.

The purification of lovastatin by the new processes of this invention is now illustrated with reference to the following Examples, to which this invention is not limited.

### Example-1

### Purification of lovastatin

50g of impure lovastatin (assay 96.33%) was added to a mixture of methanol (20ml) and toluene (180ml) under stirring at room temperature, resulting suspension was heated to 65-70° C to dissolve the solid, activated charcoal (2.5g) was added to the clear solution, stirred further for 60 minutes at 65-70° C followed by hot filtration, filtrate cooled to 0-5° C and stirred at 0-5° C for four hours, solid filtered washed with toluene and dried to furnish (36g) pure lovastatin having a chromatographic purity 99.57%, assay 99.85%, dimmer impurity 0.11% and other individual impurity 0.09%

| S.N. | Input | | | | | Output | | | | |
|---|---|---|---|---|---|---|---|---|---|---|
| | Qty. (g) | Purity (%) | SMI (%) | Imp-D (%) | Assay (%) | Qty. (g) | Purity (%) | SMI (%) | Imp-D (%) | Assay (%) |
| 2 | 50.0 | 98.74 | 0.18 | 0.26 | 96.23 | 36.0 | 99.57 | 0.09 | 0.11 | 99.85 |

| | | | | | | | | | | |
|---|---|---|---|---|---|---|---|---|---|---|
| SMI = Single maximum impurity | | | | | | | | | | |

### Example-2

### Purification of lovastatin

250g of impure lovastatin (assay 96.33%) was added to a mixture of methanol (100ml) and toluene (900ml) under stirring at room temperature, resulting suspension was heated to 65-70° C to dissolve the solid, activated charcoal (12.5g) was added to the clear solution, stirred further for 60 minutes at 65-70° C followed by hot filtration, filtrate cooled to 0-5° C and stirred at 0-5° C for four hours, solid filtered washed with toluene and dried to furnish (36g) pure lovastatin having a chromatographic purity 99.62%, assay 99.36%, dimmer impurity 0.11% and other individual impurity 0.09%.

| S.N. | Input | | | | | Output | | | | |
|---|---|---|---|---|---|---|---|---|---|---|
| | Qty. (g) | Purity (%) | SMI (%) | Imp-D (%) | Assay (%) | Qty. (g) | Purity (%) | SMI (%) | Imp-D (%) | Assay (%) |
| 1 | 250.0 | 98.80 | 0.18 | 0.28 | 96.23 | 170.0 | 99.62 | 0.09 | 0.11 | 99.36 |

| | | | | | | | | | | |
|---|---|---|---|---|---|---|---|---|---|---|
| SMI = Single maximum impurity | | | | | | | | | | |

While this invention has been described in detail with reference to certain preferred embodiments, it should be appreciated that the present invention is not limited to those precise embodiments. Rather, in view of the present disclosure, which describes the current best mode for practicing the invention, many modifications and variations would present themselves to those skilled in the art, without departing from the scope of this invention.

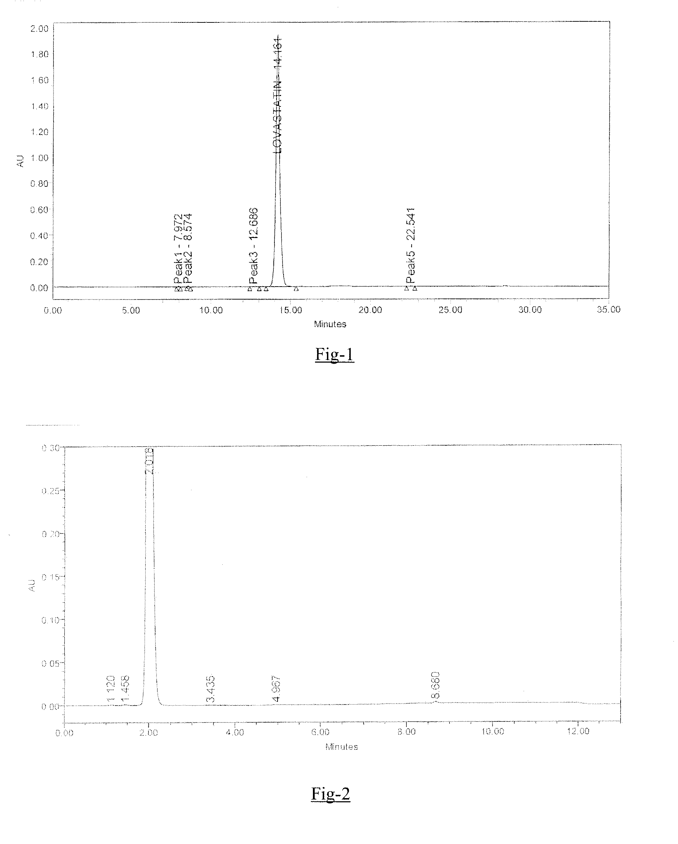

## Claims

1. A process for purification of lovastatin comprising:
dissolving lovastatin in a mixture of hydrocarbon and alcohol,
treating the solution with activated charcoal,
filtering to obtain a filtrate, and
isolating the pure lovastatin from said filtrate by crystallization,
wherein said process reduces any unknown impurities to below 0.10%.

2. The process according to claim 1, wherein the hydrocarbon is an aromatic hydrocarbon.

3. The process according to claim 2, wherein the aromatic hydrocarbon is selected from toluene, benzene and chlorobenzene.

4. The process according to claim 1, wherein the alcohol is a C₁-C₄ alcohol.

5. The process according to claim 4, wherein the alcohol is selected from methanol, ethanol and isopropanol.

6. The process according claim 1, wherein the mixture of alcohol and hydrocarbon is in the ratio of 1:9 to 9:9, preferably 1:9.

7. The process according to claim 1, wherein the temperature for the dissolution and activated charcoal treatment is room temperature to 100°C, preferably 50°C to 80°C, and more preferably 65°C to 70°C.

8. The process according to claim 1, wherein the temperature for filtration is room temperature to 100°C, preferably 50°C to 80°C, and more preferably 65°C to 70°C.

9. The process according to claim 1, wherein the temperature for the crystallization is 0°C to 40°C, preferably 0°C to room temperature, and more preferably 0°C to 5°C.

10. The process according to claim 1, wherein the time for the crystallization is 15 minutes to 12 hours, preferably 3 to 4 hours.

11. A purified lovastatin, wherein any unknown impurities in the compound is below 0.10%.
